(19) Europäisches Patentamt
European Patent Office
Office européen des brevets

(11) EP 2 705 830 A1

(12) **EUROPEAN PATENT APPLICATION**

(43) Date of publication:
**12.03.2014 Bulletin 2014/11**

(51) Int Cl.:
***A61K 8/365*** *(2006.01)* ***A61K 8/44*** *(2006.01)*
***A61Q 19/02*** *(2006.01)*

(21) Application number: **12006270.8**

(22) Date of filing: **05.09.2012**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicants:
- **Laboratoire In'Oya SAS
13120 Gardanne (FR)**
- **Aix-Marseille Université
13284 Marseille Cedex 07 (FR)**
- **Latoxan SAS
26000 Valence (FR)**

(72) Inventors:
- **Mabrouk, Kamel
13170 les pennes mirabeau (FR)**
- **Luis, José
13821 La Penne-sur-Huveaune (FR)**
- **De Pomyers, Harold
26260 Marsaz (FR)**
- **Bertin, Denis
13013 Marseille (FR)**
- **Bengeloune, Abd Haq
13090 Aix en Provence (FR)**
- **Verdoni, Marion
13010 Marseille (FR)**
- **Gigmes, Didier
13190 Allauch (FR)**

(74) Representative: **Barbot, Willy
SIMODORO
Parc Cézanne II, Bât. G
290 Avenue Galilée
13857 Aix en Provence Cedex 3 (FR)**

(54) **Use of spider venoms for skin whitening/depigmentating and composition comprising spider venoms derivatives**

(57) The present invention relates to the use of spider venom, fractions or derivatives thereof for skin whitening. The invention also relates to compositions comprising spider venom, fractions or derivatives thereof for skin whitening/depigmenting. The invention finally relates to a non-therapeutic method for whitening human skin comprising topically applying an effective amount on said human skin of the composition of the invention.

EP 2 705 830 A1

Printed by Jouve, 75001 PARIS (FR)

## Description

### FIELD OF THE INVENTION

The present invention relates to the use of spider venoms for skin whitening/depigmenting and to compositions comprising spider venoms derivatives.

### BACKGROUND OF THE INVENTION

Melanogenesis is the scientific term to refer to skin pigmentation. Although melanogenesis is necessary as a mechanism of defense against UV radiation, a disruption of this process may cause an abnormal accumulation of melanin called hyperpigmentation, including freckles, melisma and senile lentigines.

The evolution of the melanogenesis pathway takes place in the epidermis, in particular in dendritic cells, which interact with keratinocytes: melanocytes. These latter contain specific organelles called melanosomes: site of the synthesis of different pigments (melanins), responsible for our specific skin color, i.e. phototype.

Among Black people, hyperpigmentation is visualized by a spotted and uneven skin. It may be the result of immune system activity in response to inflammation, infection and/or healing but also of daily use of topical products containing substances lightening, now banned in cosmetics, such as hydroquinone and/or topical corticosteroids.

In order to reduce these dermatoses, understanding the mechanisms of melanogenesis is essential. Thus, it will be easier to visualize where the potential inhibitor will act to stop and/or reduce its production of melanin.

The melanogenic pathway is a complex process that allows the production of two different skin pigments: black-brown eumelanins and yellow to reddish pheomelanins. Tyrosinase is the key enzyme required for melanin production. Her first function is the hydroxylation of tyrosine to dihydroxyphenylalanine (DOPA), the rate-limiting step of this process. Tyrosinase allows the oxidation of DOPA to DOPAquinone. Then, two pathways can be chosen.

In the absence of thiol compounds (cysteine) (Black population), DOPAquinone is oxidized spontaneously to dopachrome, a red intermediate product. This latter can cyclize spontaneously to give 5,6-dihydroxyindole (DHI), a black insoluble molecule, or be converted into 5,6-dihydroxyindole-2-carboxylic-acid (DHICA) in the presence of a second enzyme: Tyrosinase-Related-Protein 2 (TRP-2). This second intermediate must be in the presence of a DHICA oxidase activity to be converted into DHICA-melanins. This activity is ported by Tyrosinase-Related-Protein 1 (TRP-1).

Using skin bleaching cosmetics has been a social practice for about 30 years in the black female population. Today, this concept is spreading on men in certain countries in central Africa. Currently, 60% of black African women admit to use skin lightening products in order to obtain a clearer and more uniform complexion. This practice, initiated by the media, may not only cause significant dermatological complications but also systemic complications in the long run. About 70% of users have skin problems such as keloid acne, trophic disorders, hyperpigmentation, etc. These further complications, foremost dermatologic in the first time, come from the toxic activity of the compounds present in lightener products such as hydroquinone or mercury derivatives.

Currently, in France and in Africa, the most active substances used are hydroquinone, often at high concentrations exceeding 4% and topical corticosteroids with strong activity, such as clobetasol propionate at 0.05%, which is one of the most potent topical corticosteroids. These products are used in the form of creams (hydroquinone or steroids), gels (corticosteroids) or milk (hydroquinone). The amount of active substance is often indicated but may be imprecise. The use of mercuray derivatives, previously widespread seems to be more limited today. They may be used in the form of soaps called "antiseptic".

A prospective, descriptive study has been performed over a 6-month period in Senegal including 86 female patients with a mean age of 29-34 years-old (range 16-49 years-old). The break-down by skin-bleaching products showed that topical corticosteroids were the most frequently used (78%), followed by hydroquinone (56%), products based on vegetable extracts (31,7%), caustic products (8,5%) and finally, products of unknown composition (41,4%). Two components or more are frequently combined (86.5%).

Of the 19 types of complications listed, dyschromia, including hyperpigmentation of the joints, was clearly the most common. This remains a significant stigma associated with artificial depigmentation, with 85.4% sensitivity. Striae atrophicae (72%) and skin atrophy (52.4%) were also very common, testifying to the very frequent use of corticosteroids.

Thus, there remains a need in the art for substances and cosmetic compositions able to regulate the melanogenesis pathway without side effect for black and mixed skin (phototype IV-VI).

### SUMMARY OF THE INVENTION

The inventors have focused on finding novel tyrosinase inhibitors to level the DOPA oxidase activity of tyrosinase and the DHICA oxidase activity of TRP-1. They have surprisingly found that some venoms, particularly spider venoms, contain this DOPA oxidase-inhibiting activity and DHICA oxidase-inhibiting activity. In addition, the inventors have isolated

from a venom of spider a molecule having both DOPA oxidase-inhibiting activity and DHICA-oxidase inhibiting activity.

A first object of the invention relates to a topical composition comprising spider venoms, fractions or derivatives thereof.

Another object of the invention relates to the use of spider venoms, fractions or derivatives thereof as a skin whitening/depigmenting cosmetic agent.

Another object of the invention relates to a composition comprising spider venoms, fractions of derivatives thereof as defined in the invention for treating and/or preventing hyperpigmentation such as melasma, chloasma, lentigines, vitiligo, freckles, post-inflammatory hyperpigmentation due to an abrasion, a burn, a scar, a dermatosis, a contact allergy; naevi, hyperpigmentation with a genetic determinism, hyperpigmentation of metabolic or drug origin, melanomas or any other hyperpigmentary lesions.

Finally, the invention relates to a non-therapeutic method for whitening/depigmenting human skin comprising the step of topically applying an effective amount on said human skin of spider venoms, fractions or derivatives thereof as defined in the invention.

## BRIEF DESCRIPTION OF THE DRAWINGS

Figure 1 shows the absorbance and thus the inhibiting activity obtained with 16 spider venoms.

Figure 2 shows the absorbance and thus the inhibiting activity of different fractions of V21.

Figure 3 shows the absorbance and thus the inhibiting activity of the different sub-fractions of fraction G8.

Figure 4 shows the absorbance and thus the inhibiting activity of different fractions of the venom 16.

Figure 5 shows the absorbance and thus the inhibiting activity of different fractions of the venom 4.

Figure 6 shows the IC50 curve of venom 21.

Figure 7 shows the percentage of inhibition of the DOPA oxidase activity depending on the concentration of 2,4-DHPAA.

Figure 8 shows the percentage of inhibition of the DHICA oxidase activity depending on the concentration of 2,4-DHPAA.

Figure 9 presents the absorbance, linked to the quantity of formazan produced in the cells, depending on the concentration of 2,4-DHPAA.

## DETAILED DESCRIPTION OF THE INVENTION

A first object of the invention relates to a topical composition comprising spider venoms, fractions or derivatives thereof.

As used herein, the term "topical composition" refers to a composition that is applied externally to any part of the body excluding mucous membranes such as the eyes, mouth, and so on. The topical composition may, therefore, be applied to any part of the body excluding mucous membranes such as the eyes, mouth, and so on. However, the topical composition of the invention may also be incorporated into sponges, swabs, pads and or wipes, which are the used to apply the topical composition to any part of the body excluding mucous membranes such as the eyes, mouth, and so on.

As used herein, the term "Spider venom" refers to molecules produced by spiders and injected into their victims by the means of a bite, sting or other sharp body feature, to kill or paralyze them. Such molecules of the venoms comprise but are not limited to neurotoxins, cytolysins and hemolysins.

As used herein, the term "fraction" refers to a fraction of spider venom, i.e. to a partially purified extract or compounds purified from the venom of a spider.

The term "extract" as used herein refers to a substance extracted to a natural product, regardless of the extraction method or the composition of the ingredients. For example, it includes one obtained by extracting soluble ingredients from a natural product using water or an organic solvent, or one obtained by extracting only specific ingredients, such as oil, from a natural product.

As used herein, the term "derivative" refers to any chemical or biological compound derived from an active molecule of venom. Derivatives include but are not limited to analogs.

As used herein, the term "analog" refers to a chemical compound that is structurally similar to another but differs slightly in composition. Thus an analog is a compound that is similar to or comparable in function and appearance to the reference compound.

Another object of the invention relates to the use of spider venoms, fractions or derivatives thereof as a skin whitening/depigmenting cosmetic agent.

As used herein, the term "skin whitening agent" refers to any compound or substance that have the effect of altering the pigment of the skin as long as the agent has anti-tyrosinase activity and/or anti/melanogenesis activity.

As used herein, the term "depigmenting" or "depigmentation" refers to the reduction of the pigmentation of the skin that already exists and/or also to the prevention of any additional pigmentation greater to the natural pigmentation. For example, depigmentation is obtained by reducing the formation or rate of formation of melanin.

In a preferred embodiment, the spider venoms are chosen among venoms of spiders belonging to the genus *Argiope* or *Araneus.*

According to *The World Spider Catalog 12.5,* the genus *Argiope* consists in the following species:

*Argiope acuminata, Argiope aemula, Argiope aetherea, Argiope aetheroides, Argiope ahngeri, Argiope amoena, Argiope anasuja, Argiope anomalopalpis, Argiope appensa, Argiope argentata, Argiope aurantia, Argiope aurocincta, Argiope australis, Argiope blanda, Argiope boesenbergi, Argiope bougainvilla, Argiope bruennichi, Argiope brunnescantia, Argiope buehleri, Argiope bullocki, Argiope caesarea, Argiope caledonia, Argiope cameloides, Argiope catenulata, Argiope chloreis, Argiope comorica, Argiope coquereli, Argiope dang, Argiope dietrichae, Argiope doboensis, Argiope ericae, Argiope flavipalpis, Argiope florida, Argiope halmaherensi,s Argiope intricata, Argiope jinghongensis, Argiope katherina, Argiope keyserlingi, Argiope kochi, Argiope legionis, Argiope levii, Argiope lobata, Argiope luzona, Argiope macrochoera, Argiope madang, Argiope magnifica, Argiope maja, Argiope mangal, Argiope manila, Argiope mascordi, Argiope minuta, Argiope modesta, Argiope niasensis, Argiope ocula, Argiope ocyaloides, Argiope pentagona, Argiope perforata, Argiope picta, Argiope ponape, Argiope possoica, Argiope probata, Argiope protensa, Argiope pulchello, Argiope pulchelloides, Argiope radon, Argiope ranomafanensis, Argiope reinwardti, Argiope sapoa, Argiope savignyi, Argiope sector, Argiope takum, Argiope tapinolobata, Argiope taprobanica, Argiope thai, Argiope trifasciata, Argiope truk, Argiope versicolor, Argiope vietnamensis* and *Argiope furva.*

According to *The World Spider Catalog 13.0,* the genus *Araneus* consists in the following species:

*Araneus aballensis, Araneus abeicus, Araneus abigeatus, Araneus acachmenus, Araneus aeolla, Araneus acrocephalus, Araneus acronotus, Araneus acropygus, Araneus acuminatus, Araneus acusisetus, Araneus adiantiformis, Araneus adjuntaensis, Araneus aethiopicus, Araneus aethiopissa, Araneus affinis, Araneus agastus, Araneus akakensis, Araneus aksuensis, Araneus albabdominalis, Araneus albiaculeis, Araneus albidus, Araneus albilunatus, Araneus albomaculatus, Araneus alboquadratus, Araneus albotriangulus, Aroneus alboventris, Araneus alhue, Araneus allani, Araneus alsine, Araneus altitudinum, Araneus amabilis, Araneus amblycyphus, Araneus amurius, Araneus amygdalaceus, Araneus ana, Araneus anantnagensis, Araneus anaspastus, Araneus anatipes, Araneus ancurus, Araneus andrewsi, Araneus anguinifer, Araneus angulatus, Araneus anjonensis, Araneus annuliger, Araneus annulipes, Araneus apache, Araneus apiculatus, Araneus apobleptus, Araneus appendiculatus, Araneus apricus, Araneus aragua, Araneus aralis, Araneus arenaceus, Araneus arfakianus, Araneus arganicola, Araneus argentarius, Araneus arizonensis, Araneus asiaticus, Araneus aubertorum, Araneus aurantiifemuris, Araneus auriculatus, Araneus axacus, Araneus badiofoliatus, Araneus badongensis, Araneus bagamoyensis, Araneus baicalicus, Araneus balanus, Araneus bandelieri, Araneus bantaengi, Araneus bargusinus, Araneus basalteus, Araneus bastarensis, Araneus baul, Araneus beebei, Araneus beijiangensis ,Araneus biapicatifer, Araneus bicavus, Araneus bicentenarius, Araneus bigibbosus, Araneus bihamulus, Araneus bilunifer, Araneus bimaculicollis, Araneus bimini, Araneus biprominens, Araneus bipunctatus, Araneus bispinosus, Araneus bivittatus, Araneus blaisei, Araneus blochmanni, Araneus blumenau, Araneus boerneri, Araneus boesenbergi, Araneus bogotensis, Araneus boneti, Araneus bonsallae, Araneus borealis, Araneus boreus, Araneus bosmani, Araneus bradleyi, Araneus braueri, Araneus brisbanae, Araneus bryantae, Araneus bufo, Araneus caballo, Araneus calusa, Araneus camilla, Araneus canacus, Araneus canalae, Araneus canestrinii, Araneus caplandensis ,Araneus carabellus, Araneus carchi, Araneus cardioceros, Araneus carimagua, Araneus carnifex, Araneus carroll, Araneus castilho, Araneus catillatus, Araneus catospilotus, Araneus caudifer, Araneus cavaticus, Araneus celebensis, Araneus cercidius, Araneus cereolus, Araneus chiapas, Araneus chiaramontei, Araneus chingaza, Araneus chunhuaia, Araneus chunlin, Araneus cingulatus, Araneus circe, Araneus circellus, Araneus circulissparsus, Araneus circumbasilaris, Araneus coccinella, Araneus cochise, Araneus cohnae, Araneus colima, Araneus colubrinus, Araneus compsus, Araneus comptus, Araneus concepcion, Araneus concinnus, Araneus concoloratus, Araneus corbita, Araneus corporosus, Araneus*

*corticaloides, Araneus corticarius, Araneus crinitus, Araneus crispulus, Araneus cristobal, Araneus cuiaba, Araneus cungei, Araneus cyclops, Araneus cyphoxis, Araneus cyrtarachnoides, Araneus daozhenensis, Araneus dayongensis, Araneus decaisnei, Araneus decentellus, Araneus decolor, Araneus decoratus, Araneus demoniacus, Araneus depressatulus, Araneus desierto, Araneus detrimentosus, Araneus diabrosis, Araneus diadematoides, Araneus diadematus, Araneus dianiphus, Araneus diffinis, Araneus dimidiatus, Araneus diversicolor, Araneus doenitzellus, Araneus dofleini, Araneus dospinolongus, Araneus dreisbachi, Araneus drygalskii, Araneus ealensis, Araneus eburneiventris, Araneus eburnus, Araneus ejusmodi, Araneus elatatus, Araneus elizabethae, Araneus ellipticus, Araneus elongatus, Araneus emmae, Araneus enucleatus, Araneus enyoides, Araneus excavatus, Araneus expletus, Araneus exsertus, Araneus falcatus, Araneus fastidiosus, Araneus favorabilis, Araneus faxoni, Araneus fengshanensis, Araneus ferganicus, Araneus ferrugineus, Araneus fictus, Araneus finneganae, Araneus fishoekensis, Araneus fistulosus, Araneus flagelliformis, Araneus flavisternis, Araneus flavopunctatus, Araneus flavosellatus, Araneus flavosignatus, Araneus flavus, Araneus floriatus, Araneus formosellus, Araneus frio, Araneus fronki, Araneus frosti, Araneus fulvellus, Araneus fuscinotus, Araneus gadus, Araneus galero, Araneus gazerti, Araneus geminatus, Araneus gemma, Araneus gemmoides, Araneus gerais, Araneus gestrellus, Araneus gestroi, Araneus gibber, Araneus ginninderranus, Araneus goniaeoides, Araneus goniaeus, Araneus graemii, Araneus granadensis, Araneus granti, Araneus gratiolus, Araneus groenlandicola, Araneus grossus, Araneus guandishanensis, Araneus guatemus, Araneus guerrerensis, Araneus guessfeldi, Araneus gundlachi, Araneus gurdus, Araneus guttatus, Araneus guttulatus, Araneus habilis, Araneus haematomerus, Araneus hamiltoni, Araneus hampei, Araneus haploscapellus, Araneus haruspex, Araneus herbeus, Araneus hierographicus, Araneus himalayanus, Araneus hirsti, Araneus hirsutulus, Araneus hispaniola, Araneus holzapfelae, Araneus horizonte, Araneus hortensis, Araneus hoshi, Araneus hotteiensis, Araneus huahun, Araneus hui, Araneus huixtla, Araneus humilis, Araneus idoneus, Araneus iguacu, Araneus illaudatus, Araneus indistinctus, Araneus inquietus, Araneus interjectus, Araneus inustus, Araneus iriomotensis, Araneus isabella, Araneus ishisawai, Araneus iviei, Araneus jalimovi, Araneus jalisco, Araneus jamundi, Araneus juniperi, Araneus kalaharensis, Araneus kapiolaniae, Araneus karissimbicus, Araneus kerr, Araneus kirgisikus, Araneus kiwuanus, Araneus klaptoczi, Araneus koepckeorum, Araneus komi, Araneus kraepelini, Araneus lacrymosus, Araneus ladschicola, Araneus lamperti, Araneus lancearius, Araneus lanioAraneus, lateriguttatus Araneus, lathyrinus Araneus latirostris, Araneus leai, Araneus lechugalensis, Araneus legonensis, Araneus lenkoi, Araneus lenzi, Araneus leones, Araneus liae, Araneus liber, Araneus liberalis, Araneus liberiae, Araneus licenti, Araneus lineatipes, Araneus lineatus, Araneus linshuensis, Araneus lintatus, Araneus linzhiensis, Araneus lithyphantiformis, Araneus lixicolor, Araneus loczyanus, Araneus lodicula, Araneus longicaudus, Araneus luteofaciens, Araneus lutulentus, Araneus macacus, Araneus macleayi, Araneus madagascaricus, Araneus mamillanus, Araneus mammatus, Araneus mangarevoides, Araneus margaritae, Araneus margitae, Araneus mariposa, Araneus marmoreus, Araneus marmoroides, Araneus masculus, Araneus masoni, Araneus mastersi, Araneus matogrosso, Araneus mauensis, Araneus mayumiae, Araneus mazamitla, Araneus mbogaensis, Araneus memoryi, Araneus mendoza, Araneus menglunensis, Araneus meropes, Araneus mertoni, Araneus metalis, Araneus metellus, Araneus meus, Araneus miami, Araneus microsoma, Araneus microtuberculatus, Araneus mimosicola, Araneus minahassae, Araneus miniatus, Araneus minutalis, Araneus miquanensis, Araneus missouri, Araneus mitificus, Araneus monica, Araneus monoceros, Araneus montereyensis, Araneus moretonae, Araneus mortoni, Araneus morulus, Araneus mossambicanus, Araneus motuoensis, Araneus mulierarius, Araneus musawas, Araneus myurus, Araneus nacional, Araneus nashoba, Araneus necopinus, Araneus neocaledonicus, Araneus nephelodes, Araneus nidus, Araneus nigmanni, Araneus nigricaudus, Araneus nigrodecoratus, Araneus nigroflavornatus, Araneus nigromaculatus, Araneus nigropunctatus, Araneus nigroquadratus, Araneus niveus, Araneus noegeatus, Araneus nojimai, Araneus nordmanni, Araneus nossibeus, Araneus notacephalus, Araneus notandus, Araneus noumeensis, Araneus novaepommerianae, Araneus nox, Araneus nuboso, Araneus nympha, Araneus obscurissimus, Araneus obscurtus, Araneus obtusatus, Araneus ocaxa, Araneus ocellatulus, Araneus octodentalis, Araneus octumaculalus, Araneus ogatai, Araneus omnicolor, Araneus orgaos, Araneus origenus, Araneus oxygaster, Araneus oxyurus, Araneus paenulatus, Araneus pahalgaonensis, Araneus pahli, Araneus paitaensis, Araneus pallasi, Araneus pallescens, Araneus pallidus, Araneus panchganiensis, Araneus panniferens, Araneus papulatus, Araneus partitus, Araneus parvulus, Araneus parvus, Araneus pauxillus, Araneus pavlovi, Araneus pecuensis, Araneus pegnia, Araneus pellax, Araneus penai, Araneus pentagrammicus, Araneus perincertus, Araneus petersi, Araneus pfeifferae, Araneus phaleratus, Araneus phlyctogena, Araneus phyllonotus, Araneus pichoni, Araneus pico, Araneus pictithorax, Araneus pinguis, Araneus pistiger, Araneus pius, Araneus plenus, Araneus pogisa, Araneus poltyoides, Araneus polydentatus, Araneus pontii, Araneus popaco, Araneus postilena, Araneus poumotuus, Araneus praedatus, Araneus praesignis, Araneus prasius, Araneus pratensis, Araneus principis, Araneus pronubus, Araneus prospiciens, Araneus providens, Araneus prunus, Araneus pseudoconicus, Araneus pseudosturmii, Araneus pseudoventricosus, Araneus psittacinus, Araneus pudicus, Araneus puebla, Araneus pulcherrimus, Araneus pulchriformis, Araneus punctipedellus, Araneus pupulus, Araneus purus, Araneus qianshan, Araneus quadratus, Araneus quietus, Araneus quirapan, Araneus rabiosulus, Araneus radja, Araneus ragnhildae, Araneus rainbowi, Araneus ramulosus,*

*Araneus rani, Araneus rarus, Araneus raui, Araneus recherchensis, Araneus relicinus, Araneus repetecus, Araneus riveti, Araneus roseomaculatus, Araneus rotundicornis, Araneus rotundulus, Araneus royi, Araneus rubicundulus, Araneus rubripunctatus, Araneus rubrivitticeps, Araneus rufipes, Araneus russicus, Araneus ryukyuanus, Araneus saccalava, Araneus saevus, Araneus sagicola, Araneus salto, Araneus sambava, Araneus santacruziensis, Araneus santarita, Araneus savesi, Araneus schneblei, Araneus schrencki, Araneus scutellatus, Araneus scutifer, Araneus scutigerens, Araneus selva, Araneus seminiger, Araneus senicaudatus, Araneus separatus, Araneus septemtuberculatus, Araneus sericinus, Araneus sernai, Araneus shunhuangensis, Araneus sicki, Araneus simillimus, Araneus singularis, Araneus sinistrellus, Araneus sinuosus, Araneus sogdianus, Araneus spathurus, Araneus speculabundus, Araneus sponsus, Araneus squamifer, Araneus stabilis, Araneus stella, Araneus stolidus, Araneus strandiellus, Araneus striatipes, Araneus strigatellus, Araneus strupifer, Araneus sturmi, Araneus suavis, Araneus subflavidus, Araneus subumbrosus, Araneus sulfurinus, Araneus svanetiensis, Araneus sydneyicus, Araneus sylvicola, Araneus taigunensis, Araneus talasi, Araneus talca, Araneus talipedatus, Araneus tambopata, Araneus tamerlani, Araneus taperae, Araneus tartaricus, Araneus tatianae, Araneus tatsulokeus, Araneus tellezi, Araneus tenancingo, Araneus tenerius, Araneus tengxianensis, Araneus tepic, Araneus tetraspinulus, Araneus texanus, Araneus thaddeus, Araneus thevenoti, Araneus thorelli, Araneus tiganus, Araneus tijuca, Araneus tinikdikitus, Araneus titirus, Araneus toma, Araneus tonkinus, Araneus toruaigiri, Araneus transversivittiger, Araneus transversus, Araneus triangulus, Araneus tricoloratus, Araneus trifolium, Araneus trigonophorus, Araneus triguttatus, Araneus tschuiskii, Araneus tsurusakii, Araneus tubabdominus, Araneus tuscarora, Araneus ubicki, Araneus unanimus, Araneus uniformis, Araneus unistriatus, Araneus urbanus, Araneus urquharti, Araneus ursimorphus, Araneus uruapan, Araneus urubamba, Araneus usualis, Araneus uyemurai, Araneus variegatus, Araneus venatrix, Araneus ventricosus, Araneus ventriosus, Araneus vermimaculatus, Araneus villa, Araneus vincibilis, Araneus viperifer, Araneus virgunculus, Araneus virgus, Araneus viridisomus, Araneus, viridiventris, Araneus viridulus, Araneus v-notatus, Araneus volgeri, Araneus vulpinus, Araneus vulvarius, Araneus walesianus, Araneus washingtoni, Araneus wokamus, Araneus woodfordi, Araneus workmani, Araneus wulongensis, Araneus xavantina, Araneus xianfengensis, Araneus xizangensis, Araneus yadongensis, Araneus yapingensis, Araneus yasudai, Araneus yatei, Araneus yuanminensis, Araneus yukon, Araneus yunnanensis, Araneus yuzhongensis, Araneus zapallar, Araneus zebrinus, Araneus zelus, Araneus zhangmu, Araneus zhaoi, Araneus zuluanus, Araneus zygielloides, Araneus absconditus, Araneus aethus, Araneus beipiaoensis, Araneus carbonaceous, Araneus cinefactus, Araneus defunctus, Araneus delitus, Araneus emertoni, Araneus exustus, Araneus kinchloeae, Araneus inelegans, Araneus leptopodus, Araneus liaoxiensis, Araneus longimanus, Araneus longipes, Araneus luianus, Araneus meeki, Araneus molassicus, Araneus nanus, Araneus piceus, Araneus reheensis, Araneus ruidipedalis, Araneus troschelii* and *Araneus vulcanalis.*

In a preferred embodiment, the spider venoms are chosen among *Argiope Lobata, Argiope Bruennichi* and *Araneus tartaricus* venoms.

*Argiope lobata* is a species of spider belonging to the family of Araneidae. It has a wide distribution encompassing the whole Africa and stretching to southern Europe and into Asia.

*Argiope bruennichi* is a species of spider belonging to the family of Araneidae. Its distribution encompasses central and northern Europe, northern Africa and parts of Asia.

*Araneus tartaricus* is a species of spider belonging to the family of Araneidae. It is found in western Asia.

In a preferred embodiment, the spider venom of the invention is the venom of *Argiope Lobata.*

In a preferred embodiment, the spider venom derivative of the invention is represented by formula (1).

R1- R'2 (1)

wherein

- R1 is

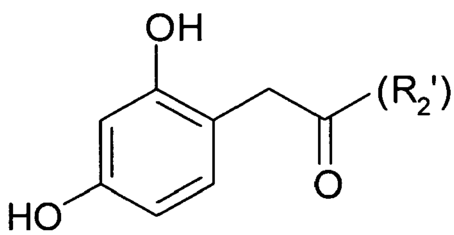

;

and

- R'2 is selected in the group comprising -OH and a substituted or unsubstituted polyamine.

Advantageously, the spider venom derivative of the invention is represented by formula (1').

R1-R2-R3-R4-R5-R6 (1')

wherein

- R1 is

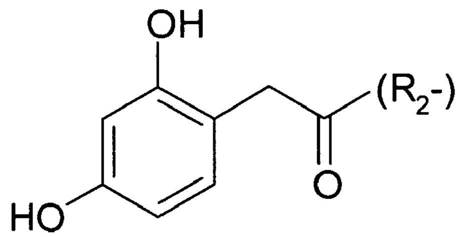

;

- R2 is -NH-CH[$(CH_2)_n$-C(=O)-$NH_2$]-C(=O)-, with n=1 or 2, preferably n=1 (i.e. R2 is asparagine);
- R3 is -NH- $(CH_2)_{n'}$-NH-, with n' is an integer between 1 and 7, preferably between 1 and 6, and most preferably n'=5;
- R4 is -NH- $(CH_2)_{n''}$-NH-, with n" is an integer between 1 and 7, preferably n"=3 or 4, and still preferably n"=3;
- R5 is -NH- $(CH_2)_{n'''}$-NH-, with n" is an integer between 1 and 7, preferably n"=3 or 4, and still preferably n"=3;
- R6 is an arginine residue; and
- R1, R2, R3, R5 or R6 can be modify so that each peptide link R1 - R2, R2 - R3 or R5 - R6 may be independently replaced by a bond selected in the group comprising -CH2-CH2-, -CH = CH-, -C(=O)-CH2-, -CH2-S-, -CH2-NH, -CH2-O-, -CH (OH)-CH2-, or -CH2-SO-.

In another preferred embodiment, the spider venom derivatives are selected in the group comprising:

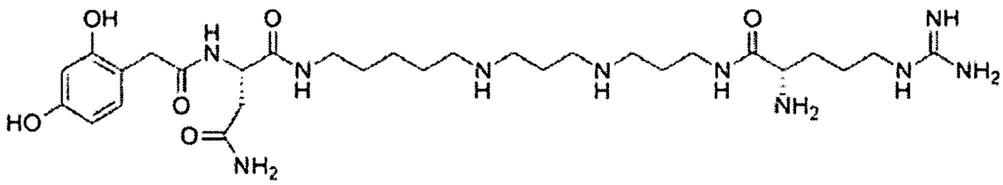

(2)

Said compound of formula (2) called Argiotoxin-636, also known as argiopine, is a polyamine isolated from the venom of A. *lobata.* (CHEMBL 1098240, CHEBI 724404).
and
2,4-Dihydroxyphenylacetic acid (2,4-DHPAA), represented by formula (3)

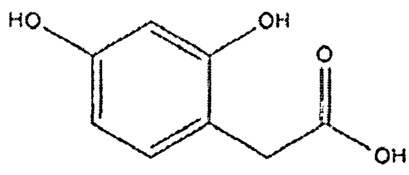

(3)

2,4-dihydroxyphenylacetic acid (2,4-DHPAA, $C_8H_8N_4$) is an aromatic molecule corresponding to the aromatic portion of argiotoxin-636.

In a preferred embodiment, the use of the whitening/ depigmenting cosmetic agent of the invention is for preventing and/or treating photo-induced or chronologic signs of aging of the skin.
As used herein, the term "photo-induced signs of aging" refers to the extrinsic aging of the skin, caused by the sun and more particularly by ultraviolet rays, which induce an increase in free radicals and oxidative stress in the dermis.
As used herein, the term "chronologic signs of aging" refers to intrinsic aging of the skin, caused by genetic and metabolic and leading to a progressive atrophy and degeneration of the dermis, hypodermis and support structures of the skin.
Another object of the invention relates to a composition comprising spider venoms, fractions of derivatives thereof as defined in the invention for treating and/or preventing hyperpigmentation such as melasma, chloasma, lentigines, vitiligo, freckles, post-inflammatory hyperpigmentation due to an abrasion, a burn, a scar, a dermatosis, a contact allergy; naevi, hyperpigmentation with a genetic determinism, hyperpigmentation of metabolic or drug origin, melanomas or any other hyperpigmentary lesions.
As used herein, the term "treating" means to cure an already present disease state or condition in a patient or subject. Treating can also include arresting the development of a disease state or condition, and relieving or ameliorating, i.e. causing regression of the disease state or condition.
The term "preventing", as used herein, means to completely or almost completely stop a disease state or condition from occurring in a patient or subject, especially when the patient or subject is predisposed to such or at risk of contracting a disease state or condition. Preventing can also include arresting the development of a disease state or condition.
As used herein, the term "hyperpigmentation" refers to a range of skin disorders caused by an increased production of melanin and results in localized areas of increase skin pigmentation. Hyperpigmentation can refer to regional hyperpigmentation due to melanocytic hyperactivity, such as idiopathic melasma, to localized hyperpigmentation due to benign melanocytic hyperactivity and proliferation, such as senescent pigmentary blemishes (senile lentigo), and to accidental hyperpigmentation, such as photo-sensitization or cicatricial hyperpigmentation, and for the treatment of certain leukodermias, such as vitiligo.
Finally, the invention relates to a non-therapeutic method for whitening/depigmenting human skin comprising the step of topically applying an effective amount on said human skin of spider venoms, fractions or derivatives thereof as defined in the invention.
As used herein, the term "effective amount" of a composition comprising an active agent means a sufficient amount of said composition to provide the desired effect.
As used herein, the term "therapeutic effect" refers to the inhibition of the abnormal condition. The term "therapeutic effect" also refers to the inhibition of factors causing or contributing to the abnormal condition. A therapeutic effect relieves to some extent one or more of the symptoms of the abnormal condition.
The followings experiments are offered to illustrate embodiments of the invention and should not be viewed as limiting the scope of the invention.

## EXAMPLES

### I. MATERIAL AND METHODS

#### a) Venom fractionation

Venoms to test were fractionated by HPLC.
Solvents were purchased from CARLO ERBA and venoms are obtained thanks to LATOXAN.
The first step is the fractionation of venoms to obtain twenty equivalent fractions in order to localize an eventual activity and identify the active molecule. 2mL of venoms solutions at 7mg/mL are manually injected after blend and filtration on MILLIPORE MILLEX-GN, Nylon 0.2μm. HPLC used is an HPLC Kontron Instruments, the absorbance is measured by UV-VIS detector at 214nm and the column is a reversed phase column (EUROSPHER 100 or 300-5 C18, 120*16mm). Method HPLC has two solvents:

- Solvent A is HPLC water + 0.1% TFA and
- solvent B is Acetonitrile 90% + HPLC water 10% + 0.1% TFA;

The flow is 4mL/min and the method can go from 0 to 60% of solvent B in 60min then 60 to 100% of solvent B in 3min.
This step must be repeated five times. Each even fractions should be pooled together to obtain more product.
When the first step is closed, all fractions were tested spectrophotometrically to localize the eventual DOPA oxidase-inhibiting activity. For this, lyophilized fractions were purified. The method used was the same that previously

described but the flow rate of 3mL/min.

### b) Mushroom tyrosinase assay

Mushroom *(Agaricus bisporus)* tyrosinase (SIGMA-ALDRICH, T3824) has both DHICA oxidase and DOPA oxidase activity.

The effect of different venoms, synthetic molecules and peptides on the tyrosinase activity was determined

#### i. Quantification of DOPA oxidase-inhibiting activity by spectrophotometry

The effect of different venoms, synthetic molecules and peptides on the DOPA oxidase activity of tyrosinase, was determined spectrophotometrically. The DOPA oxidase-inhibiting activity of tyrosinase was determined using L-DOPA as substrate. The quantity of Dopachrome (red pigment) formed was measured against blank at 475nm.

The DOPA oxidase-inhibiting activity of tyrosinase was calculated as follow:

$$\text{Inhibition percentage} = [(A-B)/(C-D)*100]$$

With:

- A : Absorbance at 475nm of the reaction medium + inhibitor sample solution with enzyme
- B : Absorbance at 475nm of the reaction medium + inhibitor sample solution without enzyme (blank)
- C : Absorbance at 475nm of the reaction medium enzyme without inhibitor sample solution
- D : Absorbance at 475nm of the reaction medium without both enzyme and inhibitor sample solution

#### ii. Quantification of DHICA oxidase-inhibiting activity by MBTH assay

This protocol was developed to visualize the DHICA oxidase-inhibiting activity, using different venoms, synthetic molecules, peptides... The assay of DHICA oxidase-inhibiting activity (in vitro) is performed by UV spectrophotometry.

The substrate DHICA is transformed to DHICA quinone complex, which is trapped by the MBTH, placed initially in excess in the reaction medium.

MBTH was obtained from ALFA AESAR and DHICA was prepared according to ITO & WAKAMATSU (1988).

DHICA oxidase was measured by the MBTH assay. MBTH captures the indole-quinone generated by the oxidation of dihydroxyindole compounds. It would be expected that if DHICA is being converted to indole-5,6-quinone-2-carboxylic acid by DHICA oxidase activity of mushroom tyrosinase, the indole product would be trapped by MBTH and the production of hydrazone-quinone adduct (IQCA-MBTH) could be detected by spectrophotometry at 492nm. (OLIVARES *et al.* (2001))

The DHICA oxidase-inhibiting activity using mushroom tyrosinase was assayed in vitro conditions following the procedure described by (WINDER & HARRIS (1991)) for DOPA oxidase with minor modifications.

In a 96-well plate, mixed 100μL of DHICA solution at 0.5mM (in 1mM EDTA solution), 50μL MBTH (3-methyl-2-benzothiazolinone)-buffer solution (5mM MBTH, 2.5% N,N-dimethylformamide, 125mM potassium phosphate, pH7), 2μL of a solution at 1mg/mL mushroom tyrosinase (added last), different volumes of inhibiting solutions depending on inhibitors tested and completed the volume final to 200μL, using a phosphate buffer solution, pH7. The solutions were assayed at different concentrations: V21 (1μL), V21 (0.4μL), AXOLIGHT (1%), AXOLIGHT (2%), L (20μM), spermine (200μM), spermidine (200μM), $PaSSO_3Ca$ (10%), $PaSSO_3Ca$ (2%) + AXOLIGHT (2%), kojic acid (10, 25 and 50μM).

Each experiment was performed in triplicate. The absorbance is measured at 492nm every minute for 15min, 30min and 60min, using a UV-VIS spectrophotometer. The blank used was the sample without the enzyme solution.

### c) Purification of the fractions

After separating the fractions into sub-fractions by the HPLC method described above, fractions were' purified by HPLC (ALLIANCE 2695 purchased from WATERS with a 2998 PDA Detector two different wavelengths 214 and 280nm).

### d) Mass MALDI-TOF

After purification, 1 μl of supernatant was spotted onto a MALDI-TOF MTP 384 target plate (BRUKER DALTONIK). Two deposits were made for each fraction. The preparation was overlaid with 1 μL of matrix solution [saturated solution of α-HCCA (alpha-cyano-4-hydroxycinnamic acid) in 50% AN and 2.5% TFA]. The matrix-sample was crystallized by air drying at room temperature for 5 minutes.

Measurements were performed with an AUTOFLEX II mass spectrometer (BRUKER DALTONIK) equipped with a 337-nm nitrogen laser. Spectra were recorded in the positive linear mode. Each spectrum was obtained at 400 nm and the acquisition time ranged from 30 to 60 seconds per spot. The method of identification included the mass to charge ratio (m/z) from 0 to 10 kDa.

**e) MTT cell viability assay**

This assay is based on the reduction of the tetrazolium ring of water soluble MTT (3-[4,5-dimethylthiazol-2-yl]-2,5-diphenyltetrazolium bromide) into insoluble purple formazan crystals by the mitochondrial succinate dehydrogenase enzyme. The amount of formazan produced is the directly related to the number of living cells, dead cells having no mitochondrial activity. MTT can thus be used to score cell viability and/or cell proliferation.

IGR39 melanoma cells were seeded at 5,000 cells/well in a 96-well plate and incubated in standard culture conditions (DMEM supplemented with 10% fetal calf serum; 37°C; 5% CO2). After 24h, the culture medium was removed and replaced with 100 μl of fresh medium containing various concentrations of 2,4-DHPAA and cells were routinely cultured for an additional period of 72h. Cells were then exposed to 50 μl of 0.5mg/ml MTT in culture medium and cultured for 2 h at 37°C. After washing with phosphate-buffered saline, 100 μl of dimethylsulfoxide were added to each well and the amount of formazan produced was evaluated by measure of the absorbance at 600 nm.

**II. RESULTS**

**a) Venoms screening**

Eighty-six venoms extracted from different species and families of venomous animals such as snakes, scorpions, spiders, etc., have been screened using UV spectrometry. The objective is to show the potential inhibiting activity of these different venoms on DOPA oxidase activity of the mushroom tyrosinase.

Venom 21, spider venom, has a DOPA oxidase-inhibiting activity of the mushroom tyrosinase. Venom 21 shows the most interesting DOPA oxidase-inhibiting activity of the 86 venoms tested.

**b) Spider venoms screening**

From this first screening, it was found that the most active venom was the *Argiope Lobata* venom (venom 21). A second screening was performed, with only spider venoms. Each initial venom solution is at 10mg/mL, and 50μL of them have been tested.

The figure 1 shows the results obtained with 16 spider venoms. Venoms 1 (A1) (which is the venom 21 of the previous screening), 4 (A4), 6 (A6), 13 (A13) and 16 (A16) show an important DOPA oxidase-inhibiting activity because they have the lowest absorbance, compared to other spider venoms. A1 (50μL) as an absorbance of about 0.1 and an inhibiting activity of DOPA oxidase of 90.9%. A4 (50μL) has an absorbance of about 0.2 and an inhibiting activity of DOPA oxidase of 74.5%. A6 (50μL) has an absorbance of about 0.3 and an inhibiting activity of DOPA oxidase of 41.0%. A13 (50μL) has an absorbance of about 0,250 and an inhibiting activity of DOPA oxidase of 56.3%. A16 (50μL) has an absorbance of about 0.175 and an inhibiting activity of DOPA oxidase of 76.5%.

Venoms 6 and 13, extracted from *Araneus Cornutus* and *Lycosa Singoriensis* do not present any reproducibility in DOPA inhibiting activity.

So, the venom 4, extracted from *Araneus tartaricus,* the venom 16, extracted from *Argiope Bruennichi* and the venom 21 extracted from *Argiope lobata* have been further studied for their activities.

**c) Fractionation of V21 *(A. lobata)*, A4 *(A. tartaricus)* and A16 *(A. Bruennichi)* venoms**

**i. Fractionation, HPLC and mass spectrometry analysis of V21 *(A. lobata)***

**Fractionation of V21**

From an initial venom solution of venom 21 at 7mg/mL, a fractionation in twenty equivalent fractions has been realized to localize this DOPA oxidase inhibition activity. These fractions are always tested using UV spectrophotometry. 40μL of each fraction were lyophilized then taken in 100μL of PBS. Only 20μL of this latter solution is used for the spectrophotometric assay.

Figure 2 shows the inhibiting activity of different fractions of V21. Fraction G8 exhibits the lowest absorbance (about 0.25) and thus the highest inhibiting activity of DOPA oxidase activity (35.6%)

These results show that the DOPA oxidase-inhibiting activity is localized in fraction G8 of venom 21.

The fractionation of the fraction G8 of the venom 21 was done by Alliance HPLC to isolate the active molecules and characterize them by mass spectrometry.

Figure 3 shows the inhibiting activity of the different sub-fractions of fraction G8. Sub-fraction 5 exhibits the lowest absorbance (between 0.3 and 0.4) and the highest inhibiting activity (between 40 and 44%). The DOPA oxidase-inhibiting activity of the fraction G8 appears to be localized in the sub-fraction 5.

**HPLC analysis of V21**

Firstly, 40μL of prefiltered venom at 7 mg/mL have been injected.

Then, after completing several fractionations under identical conditions than the prefiltered venom 21, the G8 fraction has been pooled and lyophilized.

To correlate these 2 profiles, the interest peak of the HPLC profile of the G8 fraction is the peak at 17,558 min. It corresponds to the peak at 17,921 min of the venom 21 HPLC profile.

Then, the pool of the G8 fraction was purified by semi-prep HPLC.

**Mass MALDI-TOF spectrometry analysis: characterization of the active molecule of Venom 21**

To characterize the active molecule in G8-5, a mass spectrometry MALDI-TOF analysis has been realized. All the experimentations have been realized under the same conditions: 1μL of matrix "alpha-cyano-4-hydroxycinnamic acid" with 1μL of the sample to analyze.

The molecular weight of the molecule characterized by mass spectrometry is 636,787 g/mol. In the literature, this molecule corresponds to Argiotoxine-636.

**ii. Fractionation of venom A4 *(A. tartaricus)* and A16 *(A. bruennichi)***

Under the same conditions of the venom 21, a fractionation by 3 min in semi-prep HPLC has been realized. The fractions of the venom 4 are named I2-I11 / J2-J11, and those of the venom 16 are called K2-K11 / L2-L11. The 12mL of each fraction are lyophilized and taken in 1mL of HPLC water. Then, the screening approach is the same as for the venom 21.

**Screening of venoms 16 and 4 fractions**

Screening of the venom 16 fractions has been realized using 20μL of lyophilized venom fractions solutions and taken in 1mL of HPLC water.

Figure 4 shows the absorbance and thus the inhibiting activity of different fractions of the venom 16. It appears that the fraction K6 has the lower absorbance (between 0.3 and 0.4) and the highest inhibiting activity (between 35 and 41%).

Screening of the venom 4 fractions has been realized using 60μL of venom solutions.

Figure 5 shows the absorbance and thus the inhibiting activity of different fractions of the venom 4. It appears that the fraction I7 has the lower absorbance (between 0.3 and 0.4) and the highest inhibiting activity (47%).

**Screening of venoms 16 and 4 sub-fractions**

400μL of K6 and 400μL of 17 have been fractionated by semi-prep HPLC and many sub-fractions at different times have been recovered. Each sub-fraction has been lyophilized and taken in 100μL of water HPLC.

Each active sub-fraction is undergoing characterization of the active molecule having the inhibiting activity of the DOPA oxidase activity.

**d) DOPA oxidase-inhibiting activity of V21**

**i. Half maximal inhibitory concentration of V21**

From a solution of venom 21 at 10mg/mL, a half maximal inhibitory concentration (IC50) measurement has been realized.

Figure 6 shows the IC50 curve of venom 21. This curve shows that 500μg/mL of V21 inhibits 88% of the DOPA

oxidase activity and 10μg/mL of V21 inhibits 5.6% of DOPA oxidase activity. A concentration of 62.5μg/mL of V21 inhibits 50% of DOPA oxidase activity.

**ii. Kojic acid doses-responses curve**

Doses-responses curve with kojic acid has been realized to use as reference. It permits to correlate DOPA oxidase-inhibiting activity with another inhibitor.

**e) Calibration of 2,4-DHPAA**

The 2,4-DHPAA is the aromatic portion of argiotoxine-636.

Purification of synthesized 2,4-DHPAA was done by HPLC and a calibration curve was obtained by UV spectrometry. The software which has been used is REGRESSI. Several known concentrations of DHPAA -i.e. between 0 to 1mg/mL- were obtained and stocked.

**Table 1 : Results of the calibration assay of 2,4-DHPAA**

| [DHPAA] mg/mL | Absorbances (DO) |
|---|---|
| 0.05 | 0.063 |
| 0.1 | 0.153 |
| 0.15 | 0.248 |
| 0.2 | 0.34 |
| 0.25 | 0.421 |
| 0.3 | 0.511 |
| 0.35 | 0.617 |
| 0.5 | 0.852 |
| 1 | 1.672 |

**f) Determination of IC50 of 2,4-DHPAA for the DOPA oxidase activity,**

Figure 7 shows the percentage of inhibition of the DOPA oxidase activity depending on the concentration of 2,4-DHPAA. This figure suggests that mushroom tyrosinase has two different active sites for the L-DOPA substrate with two different affinities. So, the 2,4-DHPAA would be a competitive inhibitor. Here, the activity is low, the IC50 is about 6mM, but it is not the entire molecule which has been extracted from the venom of *Argiope Lobata.* The same experiment has to be realized another time.

**g) Determination of IC50 of 2,4-DHPAA for the DHICA oxidase activity**

Figure 8 shows the percentage of inhibition of the DHICA oxidase activity depending on the concentration of 2,4-DHPAA.

The $IC_{50}$ of 2,4 DHPAA is around 0.8mg/ml or 4.8mM.

**h) Cell culture : MTT assay of 2,4-DHPAA**

This test permitted to show that synthesized 2,4-DHPAA has no toxicity.

The principle of the MTT assay is based on the metabolism of MTT by mitochondrial enzyme which is called succinate dehydrogenase. If the cells are living, the MTT crosses the cytoplasmic and is metabolized by the mitochondria.

At the end of the incubation time of cells with different concentrations of 2,4-DHPAA, cells were lysed with 100μL of DMSO. DMSO also dissolves the crystals of MTT. Then, the absorbance is read at 600 nm. The higher the DO is, the best the cell viability is and therefore the less is the cell toxicity of 2,4-DHPAA.

For this cell assay, a 96-well plate has been used. Melanosomes IGR-39 cells were used for cell viability test. The culture medium which was used is DMEM + SVF. Cells were treated with different concentrations of 2,4-DHPAA performed directly in each well, from a sterile stock solution at 10mg/mL. Incubation time was 72 hours at 37 ° C. Once this is complete, the medium was replaced by a new medium containing 0.5mg/ml MTT. Incubated 2 h at 37 ° C. Cells were washed with 100μL of PBS. After its removal, 100μL DMSO was add to realize both cells lysis and dissolution of MTT metabolized by mitochondria.

Figure 9 presents the absorbance, linked to the quantity of formazan produced in the cells, depending on the concentration of 2,4-DHPAA. For a concentration of 2,4-DHPAA comprised between 0.1 mg/mL and 2.5 mg/mL, the absorbance is comprised between 1.150 and 0.200. At 5 mg/mL of 2,4-DHPAA, the absorbance is about 0.300.

It appears that 2,4-DHPAA has no cytotoxicity in the concentration range between 0 to 5mg/mL either between 0 to 29750 μM.

## Claims

1. A topical composition comprising spider venoms, fractions or derivatives thereof.

2. Use of spider venoms, fractions or derivatives thereof as a skin whitening/depigmenting cosmetic agent.

3. The use according to claim 2, wherein spider venoms are chosen among venoms of spiders belonging to the genus *Argiope* or *Araneus.*

4. The use according to any one of claims 2 or 3, wherein spider venoms are chosen among *Argiope Lobata, Argiope Bruennichi* and *Araneus tartaricus* venoms.

5. The use according to any one of claims 2 to 4, wherein the spider venom is the venom of *Argiope Lobata.*

6. The use according to claim 2, wherein the spider venom derivative is represented by formula (1):

R1 - R'2 (1)

Wherein:

- R1 is

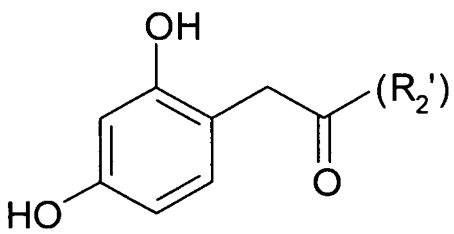

;

and

- R'2 is selected in the group comprising -OH and a substituted or an unsubstituted polyamine.

7. The use according to claim 6, wherein the spider venom derivative is represented by formula (1'):

R1-R2-R3-R4-R5-R6 (1')

wherein:

- R1 is

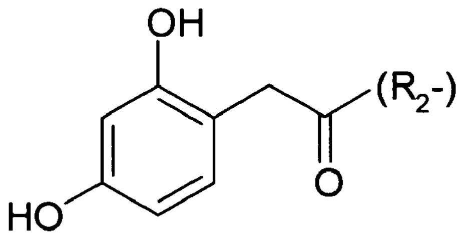

;

- R2 is -NH - CH[$(CH_2)_n$- C(=O) -$NH_2$] - C(=O) -, with n=1 or 2, preferably n=1;
- R3 is -NH- $(CH_2)_{n'}$-NH-, with n' is an integer between 1 and 7, preferably between 1 and 6, and most preferably n'=5;
- R4 is -NH- $(CH_2)_{n''}$-NH-, with n" is an integer between 1 and 7, preferably n"=3 or 4, and still preferably n"=3;
- R5 is -NH- $(CH_2)_{n'''}$-NH-, with n" is an integer between 1 and 7, preferably n"=3 or 4, and still preferably n"=3;
- R6 is an arginine residue; and
- R1, R2, R3, R5 or R6 can be modify so that each peptide link R1- R2, R2 - R3 or R5 - R6 may be independently replaced by a bond selected in the group comprising -CH2-CH2-, -CH = CH-, -C(=O)-CH2-, -CH2-S-, -CH2-NH, -CH2-O-, -CH (OH)-CH2-, or -CH2-SO-.

**8.** The use according to claim 6; wherein the spider venom derivative is selected in the group comprising:

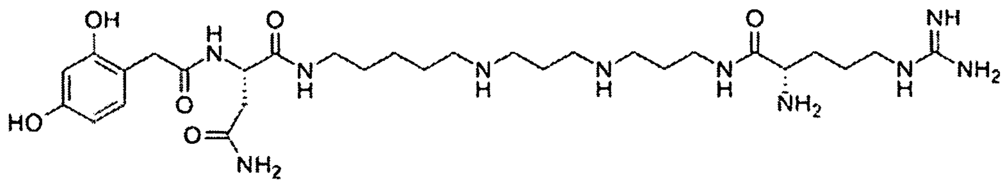

(2)

and

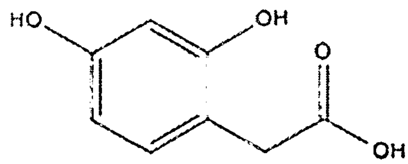

(3).

**9.** The use according to any one of claims 2 to 8, wherein said whitening/depigmenting cosmetic agent is for preventing and/or treating photo-induced or chronologic signs of aging of the skin.

**10.** A composition comprising spider venoms, fractions of derivatives thereof as defined in any one of claims 1 to 9 for treating and/or preventing hyperpigmentations such as melasma, chloasma, lentigines, vitiligo, freckles, post-inflammatory hyperpigmentations due to an abrasion, a burn, a scar, a dermatosis, a contact allergy; naevi, hyperpigmentations with a genetic determinism, hyperpigmentations of metabolic or drug origin, melanomas or any other hyperpigmentary lesions.

**11.** A non-therapeutic method for whitening/depigmenting human skin comprising the step of topically applying an effective amount on said human skin of spider venoms, fractions or derivatives thereof as defined in any one of claims 1 to 9.

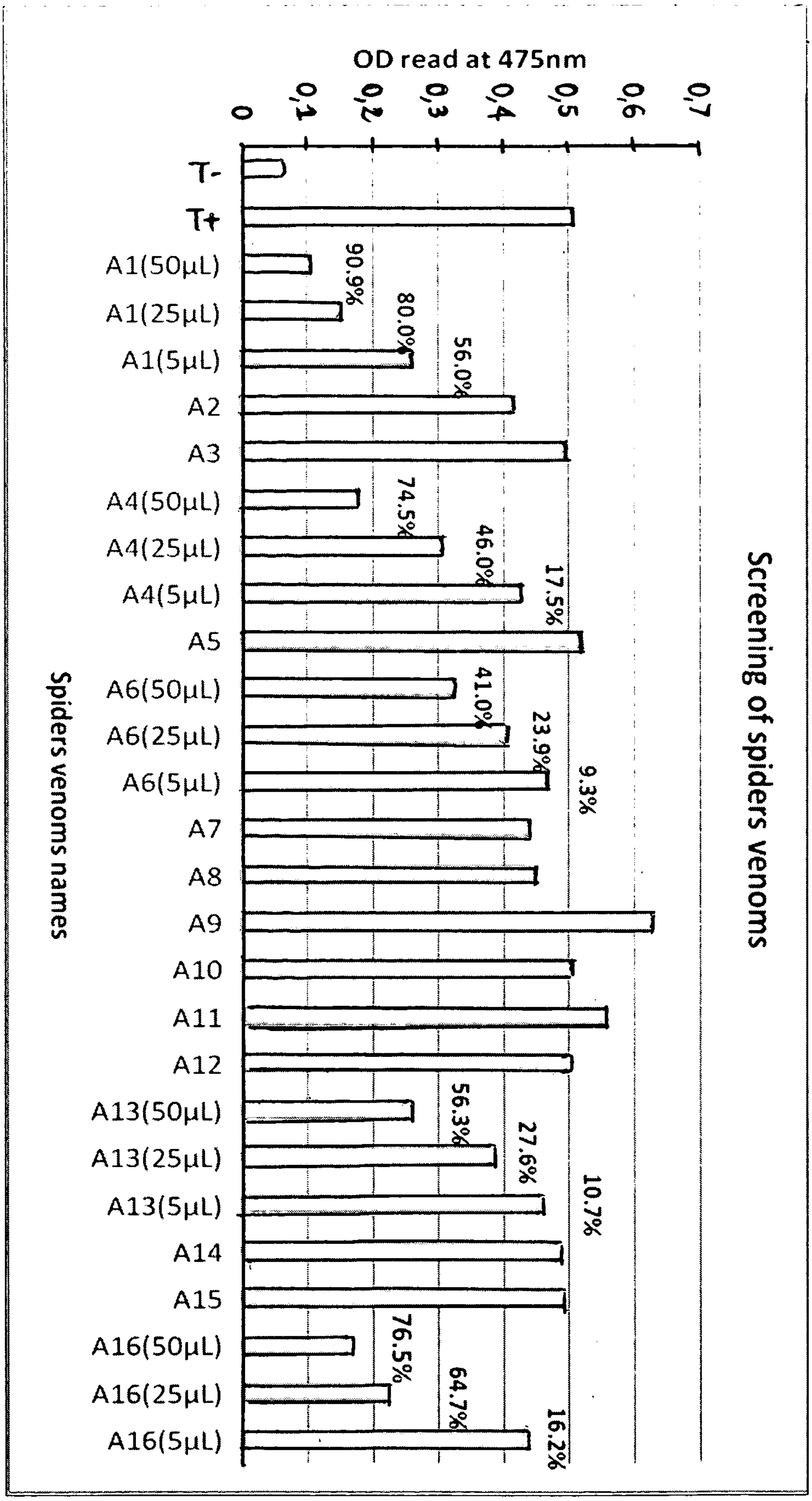
Screening of spiders venoms
OD read at 475nm
0
0,1
0,2
0,3
0,4
0,5
0,6
0,7
Spiders venoms names
T-
T+
A1(50µL)
A1(25µL)
A1(5µL)
A2
A3
A4(50µL)
A4(25µL)
A4(5µL)
A5
A6(50µL)
A6(25µL)
A6(5µL)
A7
A8
A9
A10
A11
A12
A13(50µL)
A13(25µL)
A13(5µL)
A14
A15
A16(50µL)
A16(25µL)
A16(5µL)
90.9%
80.0%
56.0%
74.5%
46.0%
17.5%
41.0%
23.9%
9.3%
56.3%
27.6%
10.7%
76.5%
64.7%
16.2%

Figure 1

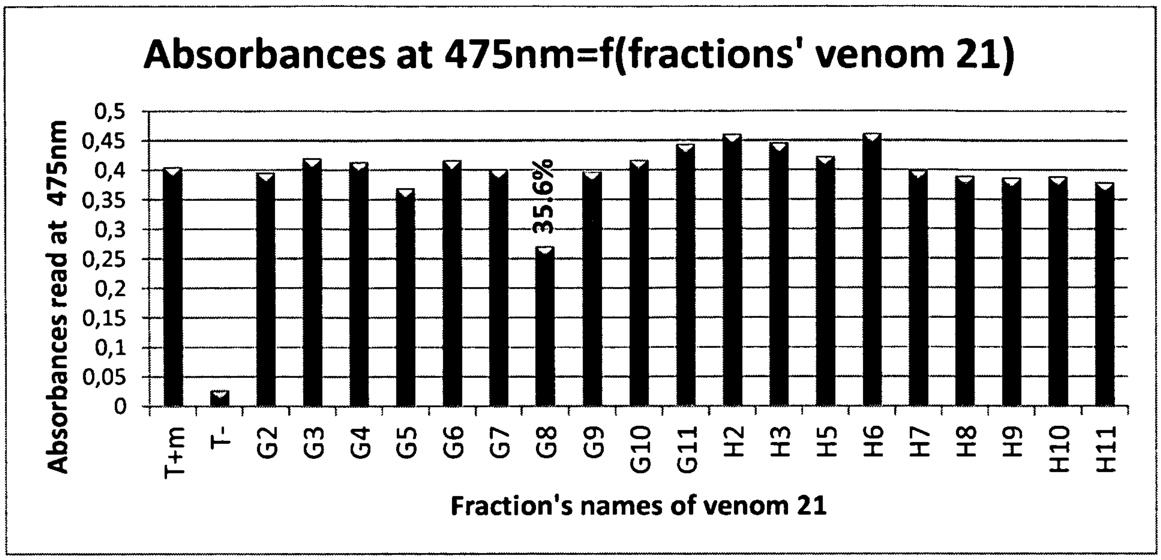


Figure 2

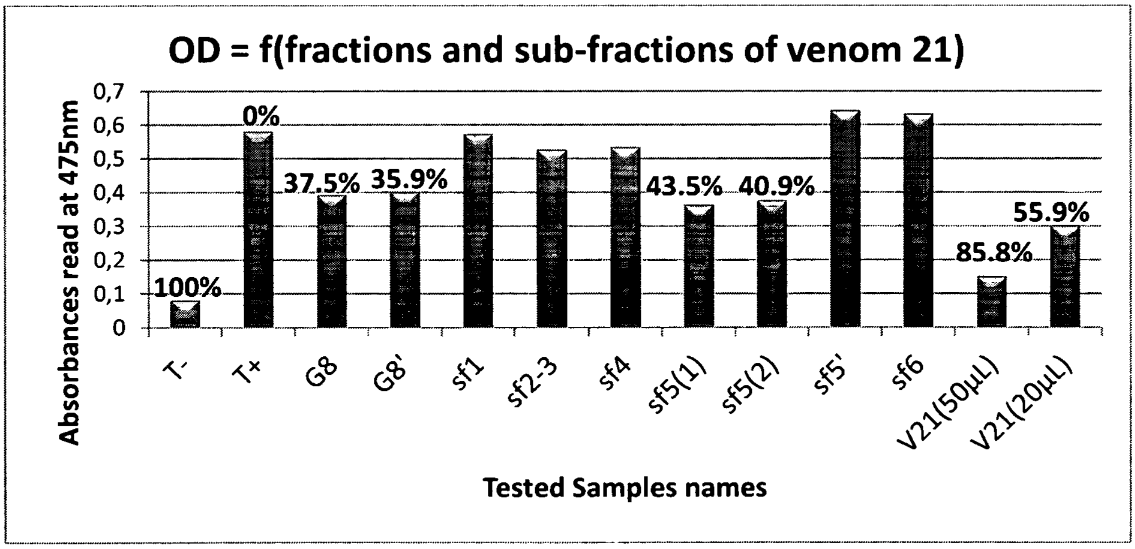


Figure 3

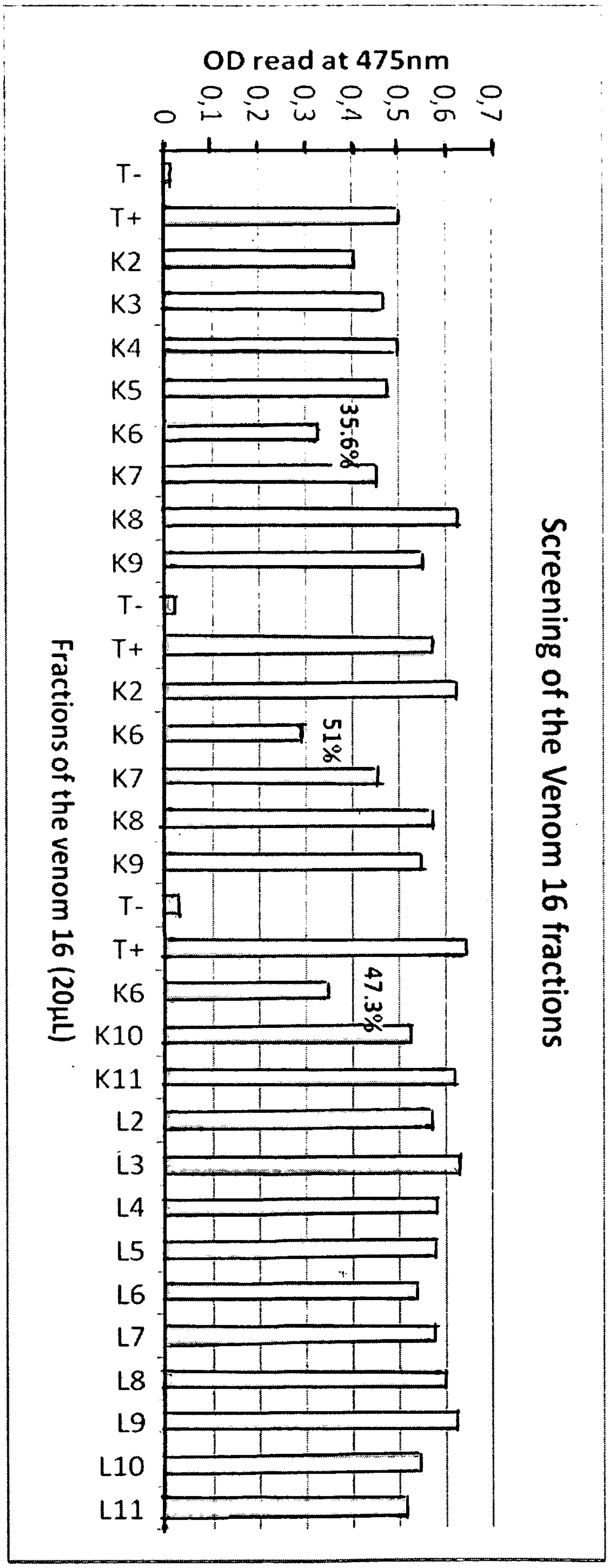
Screening of the Venom 16 fractions
OD read at 475nm
0
0,1
0,2
0,3
0,4
0,5
0,6
0,7
35.6%
51%
47.3%
T-
T+
K2
K3
K4
K5
K6
K7
K8
K9
T-
T+
K2
K6
K7
K8
K9
T-
T+
K6
K10
K11
L2
L3
L4
L5
L6
L7
L8
L9
L10
L11
Fractions of the venom 16 (20µL)

Figure 4

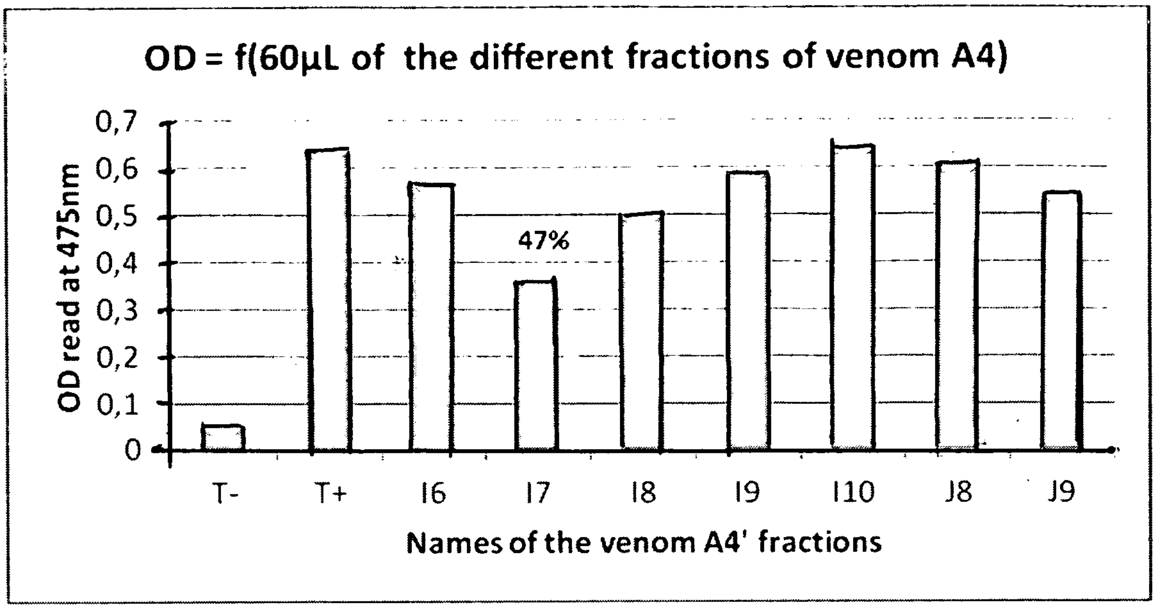


Figure 5

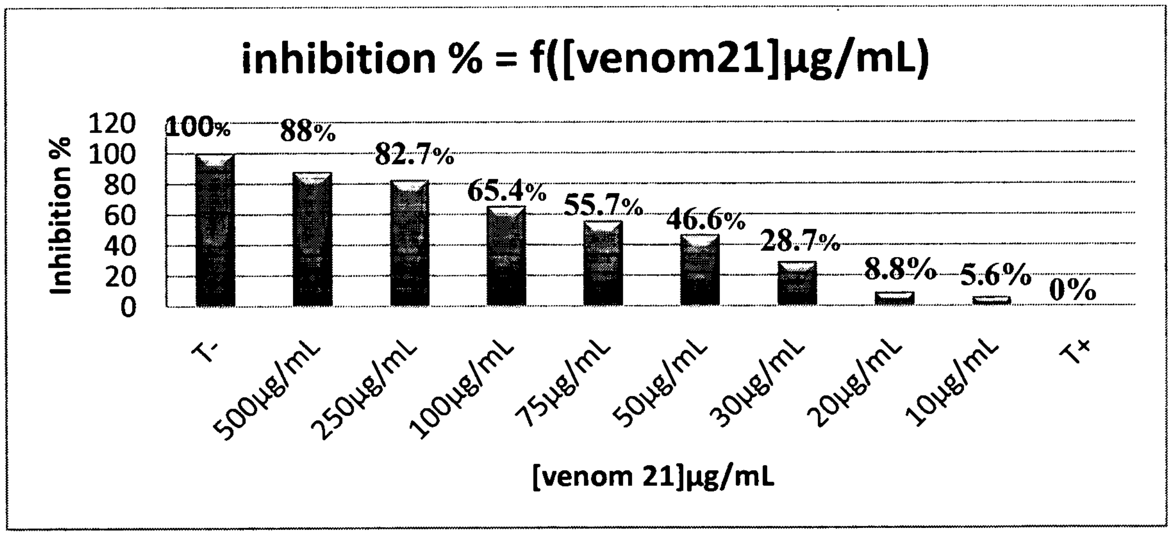


Figure 6

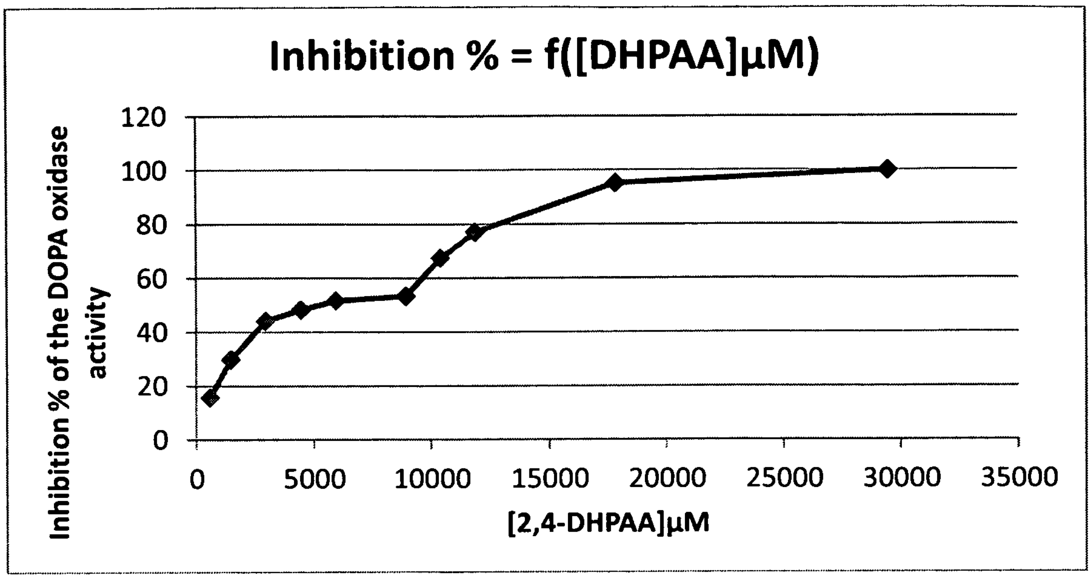


Figure 7

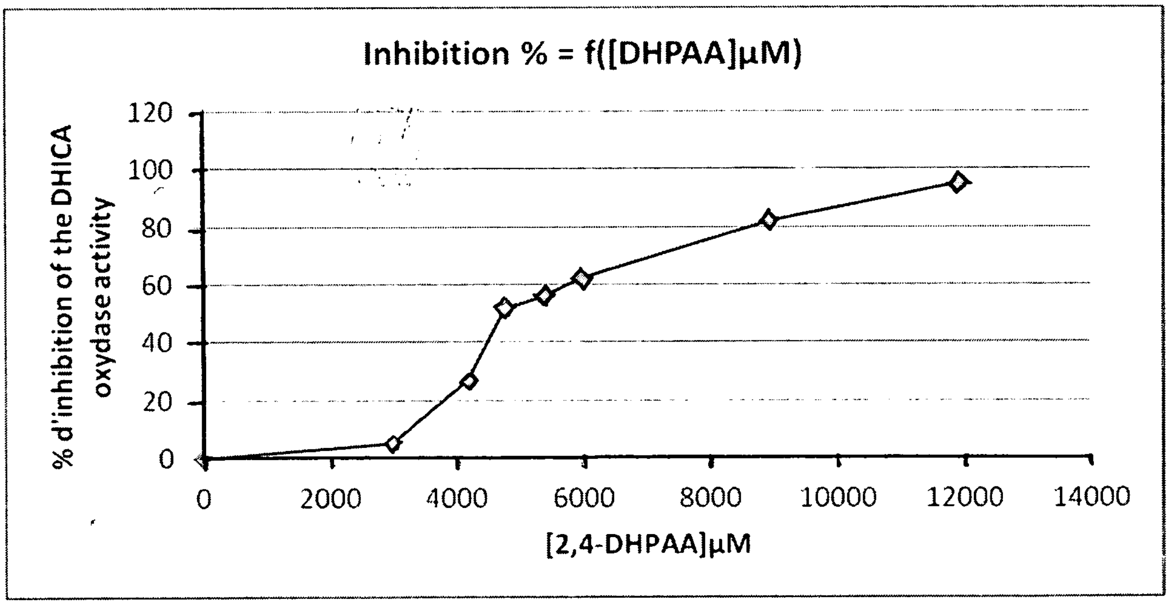


Figure 8

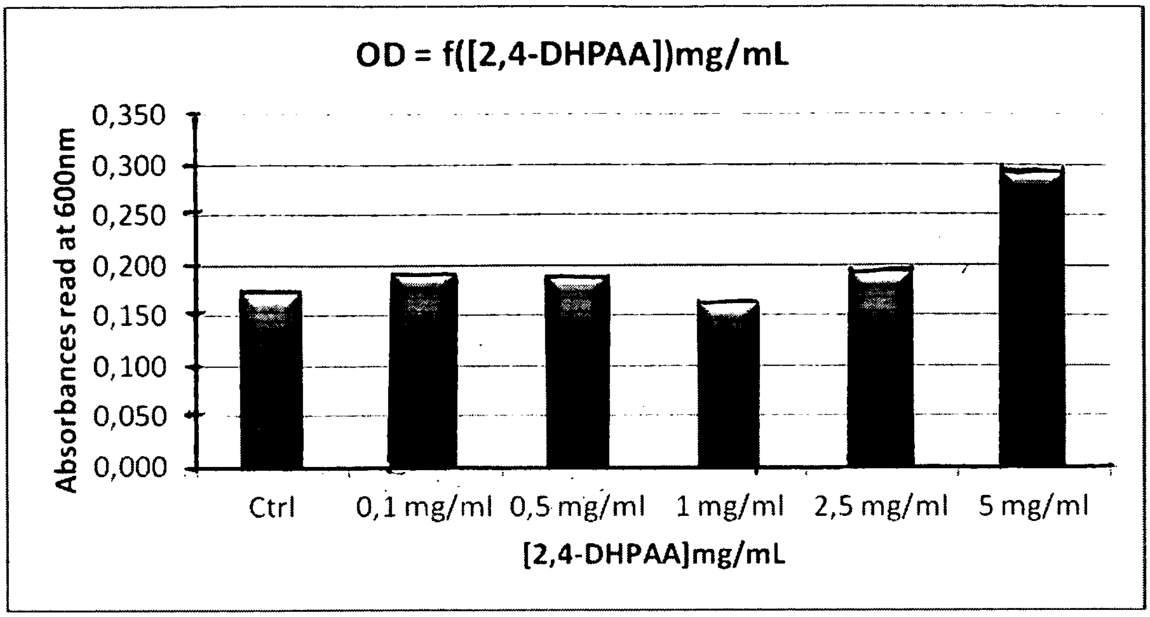
OD = f([2,4-DHPAA])mg/mL
Absorbances read at 600nm
0,350
0,300
0,250
0,200
0,150
0,100
0,050
0,000
Ctrl
0,1 mg/ml
0,5 mg/ml
1 mg/ml
2,5 mg/ml
5 mg/ml
[2,4-DHPAA]mg/mL

Figure 9

Europäisches Patentamt
European Patent Office
Office européen des brevets

# EUROPEAN SEARCH REPORT

Application Number
EP 12 00 6270

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GRISHIN E V ET AL: "Isolation and structure analysis of components from venom of the spider Argiope lobata", TOXICON, ELMSFORD, NY, US, vol. 27, no. 5, 1 January 1989 (1989-01-01), pages 541-549, XP025523944, ISSN: 0041-0101, DOI: 10.1016/0041-0101(89)90115-3 [retrieved on 1989-01-01] * page 541, line 3 - line 5 * * figure 5 * ----- | 1,10 | INV. A61K8/365 A61K8/44 A61Q19/02 |
| X | EP 0 623 339 A1 (OREAL [FR]) 9 November 1994 (1994-11-09) * claims 1,2 * ----- | 1-6,8-11 | |
| A | DATABASE CA [Online] CHEMICAL ABSTRACTS SERVICE, COLUMBUS, OHIO, US; 2011, "Bee venoms for whitening the skin and preventing and treating skin diseases", XP002696816, Database accession no. 155:202152 * * & KR 2011 0082335 A (REPUBLIC KOREA MAN RURAL DEV [KR]; DONG SUNG PHARM CO LTD [KR]) 19 July 2011 (2011-07-19) ----- | 1-11 | TECHNICAL FIELDS SEARCHED (IPC): A61K A61Q |

The present search report has been drawn up for all claims

| Place of search | Date of completion of the search | Examiner |
|---|---|---|
| Munich | 13 May 2013 | Lenzen, Achim |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

1

EPO FORM 1503 03.82 (P04C01)

## ANNEX TO THE EUROPEAN SEARCH REPORT ON EUROPEAN PATENT APPLICATION NO. EP 12 00 6270

This annex lists the patent family members relating to the patent documents cited in the above-mentioned European search report.
The members are as contained in the European Patent Office EDP file on
The European Patent Office is in no way liable for these particulars which are merely given for the purpose of information.

13-05-2013

| Patent document cited in search report | Publication date | Patent family member(s) | Publication date |
|---|---|---|---|
| EP 0623339 A1 | 09-11-1994 | DE 69404373 D1 | 04-09-1997 |
| | | DE 69404373 T2 | 22-01-1998 |
| | | EP 0623339 A1 | 09-11-1994 |
| | | ES 2105551 T3 | 16-10-1997 |
| | | FR 2704428 A1 | 04-11-1994 |
| | | JP 2696482 B2 | 14-01-1998 |
| | | JP H07316034 A | 05-12-1995 |

EPO FORM P0459

For more details about this annex : see Official Journal of the European Patent Office, No. 12/82